# EUROPÄISCHE PATENTSCHRIFT

(11) **EP 2 687 478 B1**
(45) Veröffentlichungstag und Bekanntmachung des Hinweises auf die Patenterteilung: **19.08.2015**
(21) Anmeldenummer: 13177151.1
(22) Anmeldetag: 19.07.2013
(51) Int. Cl.: B67B 1/03, B67B 3/00

(54) **Vorrichtung und Verfahren zur Behandlung von Verschlüssen für Behälter**
Method and device for treating container closures
Dispositif et procédé destinés au traitement de bouchons pour récipients

(30) Priorität: 19.07.2012 DE 102012106532
(43) Veröffentlichungstag der Anmeldung: 22.01.2014
(73) Patentinhaber: Krones AG, 93073 Neutraubling (DE)
(72) Erfinder: Laumer, Roland, 93073 Neutraubling (DE); Klepatz, Sebastian, 93073 Neutraubling (DE); Söllner, Jürgen, 93073 Neutraubling (DE)
(74) Vertreter: Nordmeyer, Philipp Werner

(56) Entgegenhaltungen:
- EP-A1- 0 017 287
- WO-A1-2009/139013
- DE-A1- 10 001 200
- DE-A1-102005 032 322
- DE-A1-102010 052 207

## Beschreibung

### Technisches Gebiet

Die vorliegende Erfindung betrifft eine Vorrichtung sowie ein Verfahren zum Behandeln von Verschlüssen für Behälter, bevorzugt zum Sterilisieren von Verschlüssen für Getränkebehälter.

### Stand der Technik

Es ist bekannt, Verschlusskappen für Behälter mittels unterschiedlicher Sterilisationsmedien zu behandeln, bevor sie auf dem jeweiligen Behälter aufgebracht, beispielsweise aufgeschraubt, werden. Im Bereich der Herstellung beziehungsweise Abfüllung von Getränken ist es beispielsweise üblich, nicht nur die Behälter selbst zu reinigen und zu sterilisieren, sondern auch die die Behälter verschließenden Verschlüsse zu reinigen und zu sterilisieren.

Besonders bei aseptischen Abfüllanlagen muss darauf geachtet werden, dass sämtliche verwendete Komponenten zur Abfüllung der jeweiligen empfindlichen Produkte steril sind, um die Produktqualität zu gewährleisten.

Eine entsprechende Reinigung der Verschlüsse kann beispielsweise dadurch vorgenommen werden, dass die Verschlüsse durch ein entsprechendes Reinigungsbad hindurchgeführt werden. Weitere Möglichkeiten zur Behandlung von Verschlüssen für Behälter ergeben sich beispielsweise durch das Ausblasen mit Sterilluft, das Besprühen mit einer Desinfektionslösung, das Spülen mit Heißluft oder mit Heißdampf, sowie das Umspülen mit einem anderen Desinfektionsbeziehungsweise Sterilisationsmedium, wie beispielsweise Wasserstoffperoxid (H₂O₂). Zweckmäßig werden die Verschlüsse auch mit ionisierter Luft behandelt um die Verschlüsse von Staubpartikeln zu reinigen.

Unterschiedliche Vorrichtungen zur Desinfizierung von Verschlüssen für Behälter sind bekannt.

Aus der DE 100 01 200 A1 ist eine Vorrichtung und ein Verfahren gemäß den Oberbegriffen der Ansprüche 1 und 12 bekannt, wobei das Verfahren zum Verschließen von Gefäßen mit einem einen wiederverschließbaren Trinkverschluss enthaltenden Verschluss und einer Schutzkappe zum Abdecken einer solchen Trinköffnung sowie eine Vorrichtung zur Durchführung dieses Verfahrens vorgesehen ist.

Beispielsweise ist aus der DE 10 2007 045 142 A1 eine Vorrichtung zum Desinfizieren von Behälterverschlüssen bekannt, bei welcher die Behälterverschlüsse auf einem innerhalb eines Gehäuses zum Desinfizieren der Behälterverschlüsse angeordneten Transportpfad geführt werden, wobei dieser Transportpfad zumindest in Abschnitten spiralförmig ist.

Aus der WO 2010/023697 A1 ist eine Vorrichtung und ein Verfahren zum Sterilisieren von Behälterverschlüssen bekannt, wobei die Behälterverschlüsse hier auf einem Transportpfad geführt werden, welcher sich in einem Gehäuse befindet, und die Wände des Gehäuses beheizbar sind.

Aus der WO 2010/023696 A1 ist ebenfalls eine Vorrichtung und ein Verfahren zum Sterilisieren von Behälterverschlüssen bekannt. Entlang des Transportpfades sind Sprühvorrichtungen vorgesehen, welche ein geheiztes und dampfförmiges Fluid auf die Behälterverschlüsse aufsprühen. Weiterhin ist ein Trocknungsmittel zum Aufbringen von Heißluft vorgesehen, und Auffangmittel zum Auffangen der Abgase aus der Passage sind vorgesehen.

Die WO 2010/103555 A1 beschreibt eine Vorrichtung und ein Verfahren zum Sterilisieren von Objekten, besonders von Verschlussdeckeln, wobei ein Injektionsmittel zum Einbringen eines Fluidflusses quer zur Transportrichtung vorgesehen ist, um eine Fluidbarriere auszubilden.

### Darstellung der Erfindung

Ausgehend von dem bekannten Stand der Technik ist es eine Aufgabe der vorliegenden Erfindung, eine Vorrichtung sowie ein Verfahren zur Behandlung von Behälterverschlüssen anzugeben, welche eine weiter verbesserte Leistung bei der Behandlung der Verschlüsse bietet.

Diese Aufgabe wird durch eine Vorrichtung mit den Merkmalen des Anspruchs 1 gelöst. Vorteilhafte Weiterbildungen ergeben sich aus den Unteransprüchen.

Entsprechend umfasst die Vorrichtung zum Behandeln von Verschlüssen für Behälter eine Behandlungskammer zum Behandeln der Verschlüsse und einen Transportpfad zum Transportieren der Verschlüsse durch die Behandlungskammer hindurch. Erfindungsgemäß umfasst der Transportpfad in der Behandlungskammer mindestens zwei separat verlaufende Verschlussführungen zum gleichzeitigen Transport von Verschlüssen.

Dadurch, dass der Transportpfad in der Behandlungskammer mindestens zwei separat verlaufende Verschlussführungen zum gleichzeitigen Transport von Verschlüssen umfasst, kann die Leistung der vorgeschlagenen Vorrichtung zur Behandlung von Verschlüssen gegenüber den im Stand der Technik genannten Sterilisierungsvorrichtungen deutlich erhöht werden. Im Stand der Technik sind die Transportpfade jeweils nur einsträngig ausgeführt, so dass eine Erhöhung der Ausbringung beziehungsweise eine Erhöhung des Durchsatzes an Verschlusskappen durch die jeweilige Sterilisationsvorrichtung zwangsläufig zu einer Verlängerung des Bauraumes der jeweiligen Behandlungskammer führt. Dies ist der Fall, da die Behandlungszeit beziehungsweise die Einwirkzeit des jeweiligen Sterilisationsmediums auf die jeweiligen Verschlüsse vorgegeben ist. Mit anderen Worten muss das Sterilisationsmedium auf den Verschluss eine bestimmte Zeit lang einwirken, um den gewünschten Abtötungseffekt zu erreichen und die Sterilisation im gewünschten Umfang zu erlangen. Bei Erhöhung der Ausbringung in den Sterilisationsvorrichtungen des Standes der Technik kann bei einer Erhöhung der Ausbringung und der damit einhergehenden Erhöhung der Transportgeschwindigkeit der einzelnen Verschlüsse die erforderliche Behandlungszeit nur durch eine Verlängerung der Behandlungskammer erreicht werden.

Durch die erfindungsgemäße Lösung hingegen kann bei einer kompakten Bauform der Behandlungskammer auch bei einer Erhöhung der Ausbringung beziehungsweise des Durchsatzes der Verschlüsse die geforderte Behandlungszeit dennoch eingehalten werden. Durch das Bereitstellen von mindestens zwei separat verlaufenden Verschlussführungen zum gleichzeitigen Transport der Verschlüsse wird nämlich die Transportgeschwindigkeit in jeder der einzelnen Verschlussführungen gegenüber einer Führung in einer einzelnen Transportführung herabgesetzt. Beispielsweise wird beim Bereitstellen von zwei nebeneinander verlaufenden, oder bevorzugt auch parallel zueinander verlaufenden, Verschlussführungen die Transportgeschwindigkeit gegenüber einer einzelnen Verschlussführung halbiert. Beim Verwenden von drei separat verlaufenden Verschlussführungen wird die Transportgeschwindigkeit entsprechend gedrittelt, usw.

Gemäß der vorliegenden Erfindung können beliebig viele separate Verschlussführungen vorgesehen sein, wobei sich jedoch eine Anordnung von 2 bis 8, bevorzugt 2 bis 5, besonders bevorzugt von 2, 3 oder 4 separaten Verschlussführungen als besonders handhabbare und zweckmäßige Anzahl anbietet.

Bevorzugt ist es, wenn sich mindestens zwei separate Verschlussführungen nebeneinander, und besonders bevorzugt parallel zueinander, erstrecken, und besonders bevorzugt linear durch die Behandlungskammer verlaufen. Durch eine parallele und lineare Ausprägung der Verschlussführungen kann eine einfache Herstellung und Justage der jeweiligen Verschlussführungen erreicht werden, bei gleichzeitig optimaler Behandlung der Verschlüsse und damit optimalem Sterilisierungsergebnis. Bei Verwendung von linear zueinander ausgebildeten Verschlussführungen kann weiterhin besonders einfach sichergestellt werden, dass die Verschlüsse jeweils vollständig vom entsprechenden Sterilisations- beziehungsweise Desinfektionsmedium umspült werden, da dann in der Behandlungskammer eine wohldefinierte Geometrie für jede Verschlussführung vorliegt und der Strom des Sterilisationsmediums entsprechend geleitet werden kann.

Bevorzugt ist eine Vorrichtung zum Einbringen eines Sterilisationsmediums in die Behandlungskammer vorgesehen, bevorzugt zum Einbringen von verdampften Wasserstoffperoxid oder Heißdampf oder verdampfte Peressigsäure, wobei die Vorrichtung zum Einbringen des Sterilisationsmediums bevorzugt an mindestens zwei Positionen das Sterilisationsmedium in die Behandlungskammer einströmt, um eine möglichst homogene Konzentration des Sterilisationsmediums in der Behandlungskammer zu erreichen.

Bevorzugt ist mindestens je eine Verschlussführung für mindestens einen ersten Verschluss und mindestens einen zweiten Verschluss vorgesehen, wobei der erste Verschluss und der zweite Verschluss unterschiedliche Dimensionen aufweisen, und bevorzugt der erste Verschluss eine Flat Cap und der zweite Verschluss eine Sport Cap sind. Auf diese Weise können mit dem gleichen System unterschiedliche Verschlusstypen flexibel verwendet werden.

Um eine möglichst umfassende und störungsfreie Umspülung der jeweiligen Verschlüsse mit dem Sterilisationsmedium zu ermöglichen, kann mindestens eine Verschlussführung eine drahtförmige Führung aufweisen, welche bevorzugt in einem Führungsrahmen gehalten ist. Die Verschlussführungen sind bevorzugt in Form von Drahtführungen ausgebildet, welche die jeweiligen Verschlüsse nur an ihren Radius halten.

Die Verschlussführungen, und bevorzugt auch die Behandlungskammer, können gegenüber der Horizontalen geneigt sein, so dass der Transport entlang des Transportpfades, und insbesondere entlang der mindestens zwei nebeneinander und bevorzugt parallel zueinander verlaufenden Verschlussführungen, im Wesentlichen auf Grundlage der Gravitation erfolgt. Mit anderen Worten "rutschen" die Verschlüsse bevorzugt von oben nach unten durch die Verschlussführungen hindurch. Die Verschlussführungen können bevorzugt unter einem Winkel von 20 und 45 Grad, besonders bevorzugt einem Winkel von 30 Grad, gegenüber der Horizontalen geneigt sein, wobei die Verschlussführungen bevorzugt im Wesentlichen parallel zueinander verlaufen.

Um den Verschlussstrom durch die Behandlungskammer hindurch steuern zu können, kann mindestens eine Steuervorrichtung zur Steuerung des Verschlussstromes in jeder Verschlussführung vorgesehen sein, bevorzugt ein Nockenrad zur Steuerung des Verschlussstromes. Besonders bevorzugt ist es, mindestens eine erste Steuervorrichtung am stromaufwärts gelegenen Ende der Behandlungskammer vorzusehen und mindestens eine zweite Steuervorrichtung an dem stromabwärts gelegenen Ende der Behandlungskammer anzuordnen, um eine vollständige Kontrolle über die in die Behandlungskammer eintretenden und die aus der Behandlungskammer austretenden Verschlüsse zu haben. Dies ist beispielsweise dann von Bedeutung, wenn die jeweilige Anlage abgefahren beziehungsweise abgeschaltet werden soll und nur noch eine begrenzte Anzahl an Verschlüssen in die Behandlungskammer eingebracht werden soll, die aber dennoch eine definierte Behandlungszeit in der Behandlungskammer erfahren sollen. Als Steuervorrichtung kann beispielsweise je ein Nockenrad pro Verschlussführung vorgesehen sein, wobei die Nocken des Nockenrades entsprechend mit den jeweiligen Verschlüssen in Eingriff gebracht werden, um deren Fluss zu steuern.

Bevorzugt ist vor mindestens einer Verschlussführung ein Sortierwerk, ein Verschlusspuffer und/oder eine Inspektionsvorrichtung zur Inspektion der Verschlüsse, bevorzugt bezüglich deren Lage und Form, vor der Behandlungskammer vorgesehen.

In einer weiteren vorteilhaften Ausführungsform ist mindestens eine Absaugkammer zum Absaugen des Sterilisationsmediums an mindestens einem Ende der Behandlungskammer vorgesehen, um ein Austreten von Sterilisierungsmedium aus der Behandlungskammer zu verhindern oder zumindest zu reduzieren, wobei bevorzugt eine erste Absaugkammer am stromaufwärts gelegenen Ende der Behandlungskammer vorgesehen ist, und bevorzugt mindestens eine zweite Absaugkammer am stromabwärts gelegenen Ende der Behandlungskammer vorgesehen ist. So kann verhindert werden, dass das Sterilisationsmedium aus der Behandlungskammer in die Umgebung austritt. Entsprechend wird das Sterilisationsmedium über die entsprechenden Absaugvorrichtungen so vollständig abgesaugt, dass ein Austritt von Sterilisationsmedium nicht stattfindet. Dabei kann besonders vorteilhaft das abgesaugte Sterilisationsmedium über bevorzugt wärmeisolierte Rohrleitungen oder Kanäle auch einer weiteren Sterilisationskammer beispielsweise zur Sterilisation von Behältervorformlingen oder Behältern zugeführt werden. Dadurch kann eine erhebliche Menge an Sterilisationsmittel eingespart werden.

In einer weiteren bevorzugten Ausführungsform schließt sich die Behandlungskammer direkt an einen Isolator, Reinraum oder Verschließraum an, in welchem das eigentliche Aufbringen der Verschlüsse auf die jeweiligen Behälter durchgeführt wird. Bevorzugt ist nach der Behandlungskammer eine Kombiniervorrichtung vorgesehen, mittels welcher der Verschlussstrom aus mindestens zwei separat verlaufenden Verschlussführungen wieder zu einem einzigen Verschlussstrom zusammengeführt wird, welcher dann der entsprechenden Verschließvorrichtung zugeführt wird. Hierzu sind bevorzugt Steuermittel derart vorgesehen, dass die Verschlüsse aus mindestens zwei separat verlaufenden Verschlussführungen abwechselnd der Verschließvorrichtung zugeführt werden.

Um eine einfache Zuführung zum Verschließer zu erreichen, können mindestens zwei Verschlussführungen am stromabwärts gelegenen Ende der Behandlungskammer zu einer einzigen Führungsrinne zusammengeführt sein.

Eine Steuervorrichtung zum Steuern des Verschlussstromes ist derart vorgesehen dass die Verschlüsse abwechselnd aus der ersten Verschlussführung und der zweiten Verschlussführung einem Verschließer zuführbar sind. Die oben gestellte Aufgabe wird weiterhin durch ein Verfahren mit den Merkmalen des Anspruchs 12 gelöst. Vorteilhafte Weiterbildungen sind in den Unteransprüchen angegeben.

Entsprechend umfasst das Verfahren zum Behandeln von Verschlüssen für Behälter die Schritte des Transportierens der Verschlüsse entlang eines Transportpfades durch eine Behandlungskammer hindurch, um die Verschlüsse zu behandeln. Erfindungsgemäß werden mindestens zwei Verschlüsse auf separaten Verschlussführungen gleichzeitig durch die Behandlungskammer transportiert.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figur 1: schematisch eine seitliche Ansicht einer Vorrichtung zum Behandeln von Verschlüssen für Behälter, wobei die seitliche Wand der Behandlungskammer fortgelassen ist;
- Figur 2: schematisch einen Querschnitt durch eine Steuervorrichtung zum Steuern des Verschlussstromes in der Vorrichtung zum Behandeln von Verschlüssen von Behältern;
- Figur 3: schematisch eine seitliche Ansicht der Steuervorrichtung der Figur 2;
- Figur 4: schematisch einen Verschließer zum Verschließen von Behältern mit Verschlüssen, welchem die Verschlüsse von der Vorrichtung zum Behandeln von Verschlüssen aus zugeführt werden;
- Figur 5: eine schematische Draufsicht auf eine aseptische Füllanlage; und
- Figur 6: einen schematischen Querschnitt durch eine weitere Vorrichtung zum Behandeln von Verschlüssen für Behälter.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet und auf eine wiederholte Beschreibung dieser Elemente wird in der nachfolgenden Beschreibung teilweise verzichtet, um Redundanzen zu vermeiden.

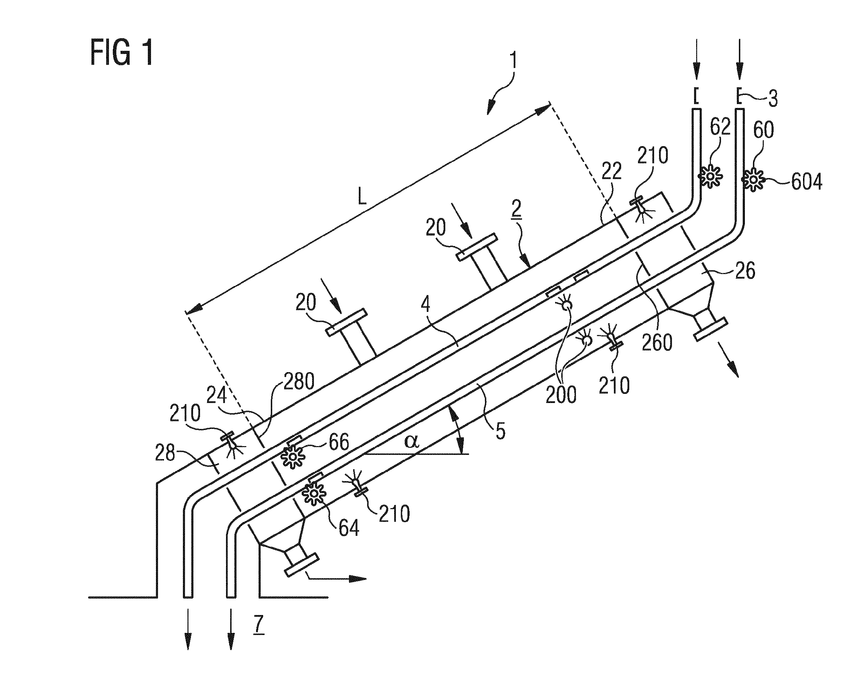

Figur 1 zeigt schematisch eine Vorrichtung 1 zum Behandeln von Verschlüssen für Behälter, wobei diese Vorrichtung 1 hier in einer Seitenansicht gezeigt ist und eine Wand einer Behandlungskammer 2 in der Abbildung so fortgelassen ist, dass ein Einblick in die Behandlungskammer 2 möglich ist. Die Behandlungskammer 2 ist im Wesentlichen gasdicht ausgebildet. Über entsprechende Anschlussflansche 20 wird ein durch die Pfeile angedeutetes Sterilisierungsmedium in die Behandlungskammer 2 eingeführt, um eine Sterilisierung des Inhaltes der Behandlungskammer 2 zu erreichen. Als Sterilisierungsmedium wird bevorzugt verdampftes Wasserstoffperoxid, welches vorzugsweise zusammen mit heißer Sterilluft in die Behandlungskammer 2 eingeblasen wird, oder Heißdampf verwendet.

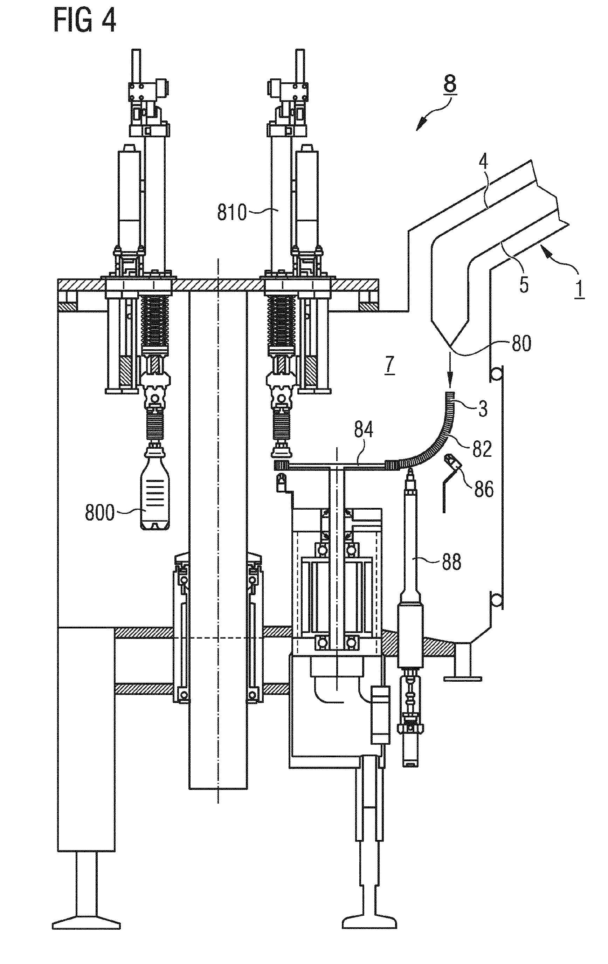

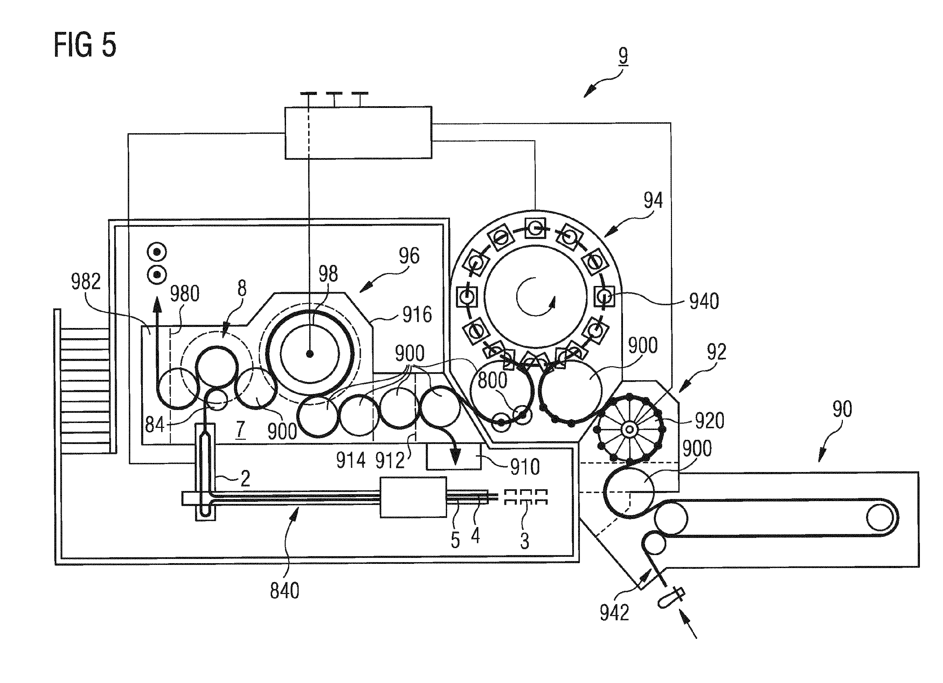

Verschlüsse 3, welche zum Verschließen von Behältern verwendet werden sollen, sind beispielsweise in Form von Flat Caps oder Sport Caps zum Aufschrauben auf PET-Flaschen bekannt. Diese Verschlüsse 3 werden den Verschlussführungen 4, 5 der Vorrichtung 1 jeweils über ein nicht gezeigtes Sortierwerk zugeführt. Die Verschlüsse 3 werden dabei am stromaufwärts gelegenen Ende 22 der Behandlungskammer 2 in die Behandlungskammer 2 eingebracht, auf dem Transportpfad durch die Behandlungskammer 2 hindurchgeführt, und am stromabwärts gelegenen Ende 24 der Behandlungskammer 2 wieder aus dieser ausgeleitet. Die Verschlüsse 3 werden dann einem Verschließer zugeführt, mittels welchem die Verschlüsse 3 auf die Behälter aufgebracht werden. Auf den Verschließer wird nachfolgend unter Bezugnahme auf die Figuren 4 und 5 weiter eingegangen werden.

Die Verschlüsse 3 werden auf zwei separat verlaufenden Verschlussführungen 4, 5 gleichzeitig durch die Behandlungskammer 2 transportiert. In weiteren Ausführungsformen können auch mehr als zwei Verschlussführungen vorgesehen sein, welche separat durch die Behandlungskammer 2 hindurch verlaufen. Mit anderen Worten bildet jede der separaten Verschlussführungen 4,5 einen separaten Strang des Verschlussstroms zum Transport der Verschlüsse 3 aus.

Unter "nebeneinander" wird hier verstanden, dass sich die separaten Verschlussführungen 4, 5 in der Behandlungskammer 2 befinden und ein Transport der Verschlüsse 3 in beiden Verschlussführungen 4, 5 im Wesentlichen in die gleiche Richtung stattfindet. Die Verschlüsse 3 werden dabei in jeder der beiden Verschlussführungen 4, 5 in einem separaten Verschlussstrom gefördert.

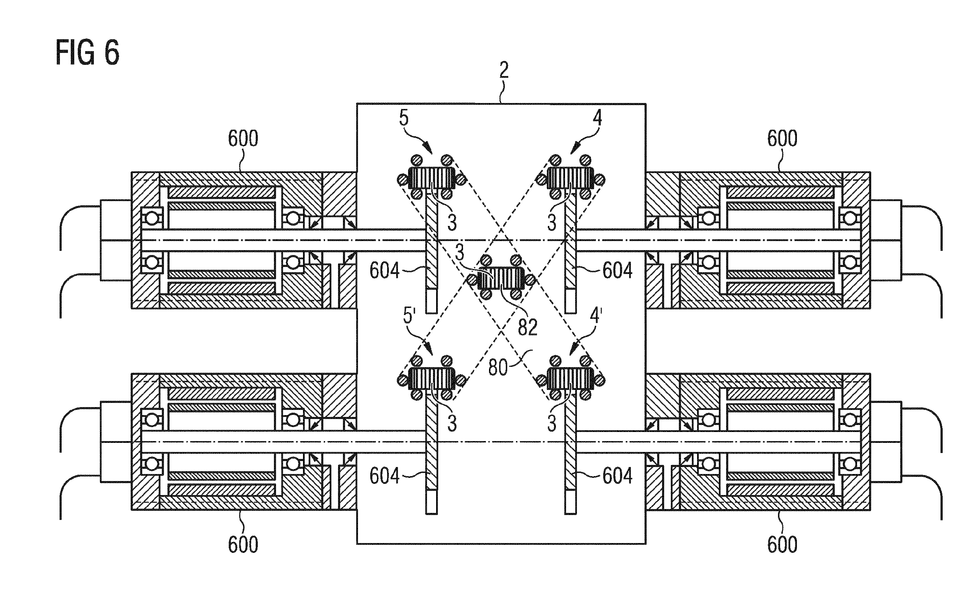

Die Verschlussführungen 4, 5 können dabei in einer beliebigen räumlichen Anordnung in der Behandlungskammer 2 angeordnet sein. Wenn mehr als zwei Verschlussführungen vorgesehen sind, können diese auch verteilt im Raum nebeneinander angeordnet sein, also beispielsweise auch auf unterschiedlichen Ebenen. Ein entsprechendes Beispiel wird nachfolgend in der Figur 6 gezeigt werden.

Dadurch, dass die Verschlüsse 3 auf den beiden separat verlaufenden Verschlussführungen 4, 5 durch die Behandlungskammer 2 hindurch transportiert werden, kann erreicht werden, dass die Transportgeschwindigkeit eines individuellen Verschlusses 3 entlang des Transportpfades geringer ist als im Stand der Technik, gemäß welchem sämtliche Verschlüsse entlang einer einzigen Verschlussführung geführt werden. Dies ist daher der Fall, da sich der vom Verschließer geforderte Verschlussstrom auf mindestens zwei separat verlaufende Verschlussführungen 4, 5 verteilt.

Auf diese Weise kann auch erreicht werden, dass die Behandlungslänge L, entlang welcher die Verschlüsse 3 mit dem jeweiligen Sterilisationsmedium in Kontakt kommen, relativ kürzer gehalten werden kann als bei den aus dem Stand der Technik bekannten Sterilisierungsvorrichtungen. Entsprechend kann die gesamte Baulänge der Vorrichtung 1 bei gleichzeitiger Erhöhung der Leistung kompakt gehalten werden.

Die Verschlussführungen 4, 5 sind, wie in der seitlichen Schnittdarstellung der Vorrichtung 1 in Figur 1 gezeigt, schräg gegenüber der Horizontalen angeordnet. Bevorzugt liegt ein Winkel α zwischen der Horizontalen und der jeweiligen Verschlussführung 4, 5 im Bereich von 20 bis 45 Grad, besonders bevorzugt bei 30 Grad. Auf diese Weise können die Verschlüsse 3 aufgrund ihrer Schwerkraft durch die Behandlungskammer 2 hindurch transportiert werden und benötigen keinen separaten Antrieb.

Der Verschlussstrom in die Behandlungskammer 2 hinein wird bevorzugt über Steuervorrichtungen 60, 62 gesteuert, welche jeweils in den Verschlussführungen 4, 5 vor der Behandlungskammer 2 angeordnet sind. Die Steuervorrichtungen 60, 62 sind bevorzugt in Form von Nockenrädern 604 vorgesehen, welche Nocken aufweisen, die jeweils mit den Verschlüssen 3 in Eingriff gebracht werden. Mittels der Steuervorrichtungen 60, 62 kann der Verschlussstrom durch die Verschlussführungen 4, 5 unmittelbar und auf den einzelnen Verschluss genau gesteuert werden. Mittels der Steuervorrichtung 60, 62 kann gleichzeitig auch die Anzahl der in die Behandlungskammer 2 eingebrachten Verschlüsse bestimmt und gesteuert werden, so dass auf diese Weise sichergestellt werden kann, dass beispielsweise beim Herabfahren beziehungsweise Ausschalten der Vorrichtung 1 nur noch so viele Verschlüsse 3 in die Behandlungskammer 2 eingebracht werden, wie auch Behälter verschlossen werden sollen. Auf diese Weise kann vermieden werden, dass nach dem Anhalten der Befüllvorrichtung noch Verschlüsse in der Behandlungskammer 2 vorliegen. Dies ist insbesondere bei der Verwendung der Vorrichtung 1 im Zusammenhang mit einer aseptischen Befüllungs- und Verschließanlage von Bedeutung, um sicherzustellen, dass beim späteren Wiederanfahren der Anlage nur Verschlüsse zum Verschließen der jeweiligen Behälter verwendet werden, welche eine definierte Sterilisierung durchlaufen haben. Jede der Verschlussführungen 4, 5 kann bevorzugt über ein vorgeschaltetes Sortierwerk so mit Verschlüssen 3 versorgt werden, dass die Verschlüsse 3 in einer korrekten Ausrichtung in die jeweilige Verschlussführung 4, 5 eingebracht werden.

Weiterhin ist es vorteilhaft, vor dem Eintritt der Verschlüsse 3 in die Behandlungskammer 2 mittels einer hier ebenfalls nicht gezeigten Inspektionsvorrichtung die korrekte Lage, Ausrichtung sowie Form der Verschlüsse 3 zu kontrollieren.

Zwischen der nicht gezeigten Inspektionsvorrichtung und dem Eintritt der Verschlüsse 3 in die Behandlungskammer 2 ist mit Vorzug auch ein ebenfalls nicht gezeigter Verschlusspuffer angeordnet, um auch kurzfristig Schwankungen des Verschlussstroms ausgleichen zu können.

Eine genaue Steuerung der Verweilzeit der jeweiligen Verschlüsse 3 in der Behandlungskammer 2 kann über zwei weitere Steuervorrichtungen 64, 66 erreicht werden, welche jeweils am stromabwärts gelegenen Ende 24 der Behandlungskammer 2 in den Verschlussführungen 4, 5 so angeordnet sind, dass sie mit jedem der Verschlüsse 3 in Eingriff kommen. Auf diese Weise kann erreicht werden, dass eine bestimmte Verweilzeit eines individuellen Verschlusses 3 in der Behandlungskammer 2 eingehalten wird, auch wenn die Behandlungskammer 2 beispielsweise zu Beginn des Prozesses zunächst leer ist und die Verschlüsse 3 aufgrund der Gravitation nach dem Einleiten mittels der Steuervorrichtungen 60, 62 direkt zum stromabwärts gelegenen Ende 24 der Behandlungskammer 2 rutschen.

Durch das Vorsehen von mindestens zwei separat verlaufenden Verschlussführungen 4, 5 kann die gesamte Baulänge der Behandlungskammer 2, und damit analog auch die Behandlungslänge L, reduziert werden, da die Transportgeschwindigkeit eines individuellen Verschlusses 3 gering gehalten werden kann. Da die Deckenhöhe in Abfüllhallen üblicherweise auf ca. 6 Meter beschränkt ist, kann auf diese Weise eine Vorrichtung 1 zur Behandlung von Verschlüssen 3 vorgesehen werden, welche bei einer hohen Ausbringung an Verschlüssen 3, beziehungsweise einer hohen Leistung, ein kompaktes Baumaß ermöglicht.

Das über die Anschlussflansche 20 zugeführte Sterilisationsmedium ist bevorzugt Wasserstoffperoxid (H₂O₂), welches verdampft wird und zusammen mit einem heißen Luftstrom, bevorzugt einem heißen Sterilluftstrom, in die Behandlungskammer 2 eingebracht wird. Neben der eigentlichen Behandlung der Verschlüsse 3 dient das so eingebrachte Sterilisationsmedium gleichzeitig auch zur Sterilisation der Behandlungskammer 2, der Wände der Behandlungskammer 2, sowie der Verschlussführungen 4, 5, in welchen die Verschlüsse 3 geführt werden.

An die Behandlungskammer 2 schließen sich in dem in Figur 1 gezeigten Ausführungsbeispiel unmittelbar Absaugvorrichtungen 26, 28 an. Über die stromaufwärts von der Behandlungskammer 2 gelegene Absaugvorrichtung 26 sowie über die stromabwärts der Behandlungskammer 2 gelegene Absaugvorrichtung 28 wird das über die Anschlussflansche 20 eingebrachte Sterilisationsmedium wieder abgesaugt. Auf diese Weise kann einerseits sichergestellt werden, dass das Sterilisationsmedium nicht in andere Anlagenbereiche oder in die Abfüllhalle gelangt. Andererseits wird eine vollständige Durchspülung der Behandlungskammer 2 mit Sterilisationsmedium sichergestellt.

Um einen geringen Verlust an Sterilisationsmedium beziehungsweise eine hohe Verweildauer des Sterilisationsmediums in der Behandlungskammer 2 und damit eine effiziente Verwendung des eingesetzten Sterilisationsmediums zu erreichen, sind bevorzugt Blenden 260, 280 zwischen der Behandlungskammer 2 und den Absaugvorrichtungen 26, 28 vorgesehen. Entsprechend wird das Sterilisationsmedium nur durch die entsprechend ausgeschnittenen Durchführungen der Verschlussführungen 4, 5 in den Blenden 260, 280 über die Absaugvorrichtung 26, 28 abgesaugt.

Die Behandlungskammer 2 mit der daran angebrachten stromabwärts gelegenen Absaugvorrichtung 28 schließt bevorzugt unmittelbar an den Raum 7 an, in welchem der Verschließer untergebracht ist und welchem die mittels der Vorrichtung 1 behandelten Verschlüsse 3 zugeführt werden sollen. Der Raum 7 ist dabei bevorzugt als Isolatorgehäuse, Reinraum oder Verschließraum ausgebildet.

Um besonders komplizierte Geometrien von Verschlüssen 3 vollständig mit dem Sterilisationsmedium zu umspülen, können bevorzugt zusätzliche Düsen 200 zum Einbringen von Sterilisationsmedium vorgesehen sein. Diese Düsen 200 sind in Figur 1 schematisch angedeutet, können aber an jeder beliebigen anderen Stelle innerhalb der Behandlungskammer 2 angeordnet sein, welche zum Spülen der jeweiligen Verschlussgeometrie sinnvoll ist.

Neben der Sterilisierung der Wände der Behandlungskammer 2 sowie der Verschlussführungen 4, 5 und sämtlicher anderer in der Behandlungskammer 2 vorgesehener Komponenten durch das Sterilisationsmedium, sind zusätzlich Reinigungsdüsen 210 vorgesehen, welche zur Reinigung der Behandlungskammer 2 und der Absaugvorrichtungen 26, 28 dienen, und diese beispielsweise mit einem Reinigungsfluid durchspülen können.

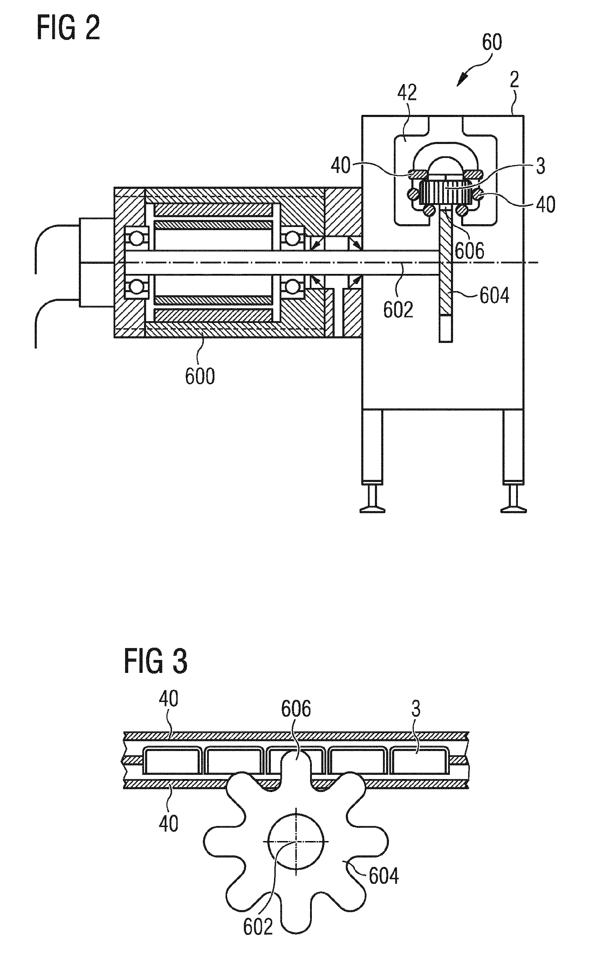

Figur 2 zeigt schematisch einen Querschnitt durch eine Steuervorrichtung, beispielsweise die Steuervorrichtung 60 aus Figur 1.

Die Steuervorrichtung 60 umfasst einen Steuermotor 600, welcher bevorzugt als Schrittmotor ausgebildet ist, und auf dessen Achse 602 ein Nockenrad 604 aufgesetzt ist. Das Nockenrad 604 greift mit einer Nocke 606 in einen Verschluss 3 ein, welcher als Verschlusskappe ausgebildet ist. Durch den Eingriff der Nocke 606 in den Verschluss 3 kann jeder einzelne Verschluss 3 kontrolliert gefördert werden und es kann auf diese Weise eine verschlussgenaue Förderung erreicht werden. In Figur 3 ist dieser Eingriffvorgang der Nocke 606 in den Verschluss 3 noch einmal schematisch in einer seitlichen Schnittdarstellung gezeigt.

Aus Figur 2 ergibt sich auch eine bevorzugte Ausbildung einer Verschlussführung, welche derart ausgebildet ist, dass der Verschluss 3 auf drahtförmigen Führungen 40, welche parallel zueinander verlaufen, geführt wird. Entsprechend liegt der Verschluss 3 an den drahtförmigen Führungen 40 nur mit sehr kleinen Kontaktbereichen auf, so dass der Verschluss 3 im Wesentlichen vollständig mit dem Sterilisationsmedium umspült wird.

Die drahtförmigen Führungen 40 werden in Führungsrahmen 42 gehalten, welche in regelmäßigen Abständen voneinander, beispielsweise an den Wänden der Behandlungskammer 2, befestigt sind.

Figur 4 zeigt in einer schematischen Seitenansicht einen Verschließer 8, welcher sich direkt an die Vorrichtung 1 zum Behandeln von Verschlüssen anschließt. Die Vorrichtung 1 schließt sich hier direkt an den Raum 7 des Verschließers 8 an, wobei der Raum 7 hier als Reinraum ausgeführt ist und der Verschließer 8 Teil einer aseptischen Abfüllanlage ist.

Die beiden Verschlussführungen 4, 5, welche aus der Vorrichtung 1 zum Behandeln von Verschlüssen herausgeführt werden, werden über eine Zusammenführeinrichtung 80 wieder zu einem einzigen Verschlussstrom zusammengefasst und nachfolgend in einer entsprechenden Führungsrinne 82 einem Einteilrad 84 des Verschließers 8 zugeführt. Über das Einteilrad 84 werden die Verschlüsse vereinzelt und auf den Abstand der zu verschließenden Behälter 800 gebracht. In der Zusammenführvorrichtung 80 werden hierfür bevorzugt, so wie auch schematisch nachfolgend in Figur 6 gezeigt, die Verschlüsse aus den unterschiedlichen Verschlussführungen 4, 5 abwechselnd in die Führungsrinne 82 eingespeist.

An der Führungsrinne 82 können weitere Düsen 86 vorgesehen sein, um Behandlungsrückstände aus den Verschlüssen 3 zu entfernen. Die Düsen 86 strömen dabei bevorzugt heiße Sterilluft auf die entsprechenden Verschlüsse 3 auf, um ein Entfernen von Behandlungsrückständen zu erreichen.

Ein Verschlussstopper 88 ist vorgesehen, mittels welchem der Strom der Verschlüsse 3 in der Führungsrinne 82 zumindest kurzzeitig unterbrochen werden kann, um beispielsweise bei erkannten Lücken im Strom der Behälter 800 eine Zuführung von Verschlüssen 3 zu den Lücken zu verhindern.

Die einzelnen Verschlüsse 3 werden über die jeweiligen Verschließelemente 810 gemäß der durch das Einteilrad 84 vorgegebenen Teilung aufgenommen und dann entsprechend zum Verschließen der Behälter 800 transportiert und auf die Behälter 800 aufgebracht, bevorzugt aufgeschraubt.

Der in Figur 4 gezeigte Verschließer 8 ist ein Schraubverschließer, welcher beispielsweise auf einer Behälterleistung von 10.000 Behältern pro Stunde eingesetzt werden kann. Andere Verschließertypen, beispielsweise Verschließer, bei denen der Verschluss aufgeklebt oder aufgeschweißt oder aufgesiegelt wird, können aber ebenfalls mit einem Verschlussstrom aus der Vorrichtung 1 versorgt werden.

Figur 5 zeigt schematisch eine aseptische Abfüllanlage 9, welche aus mehreren Modulen und Behandlungseinheiten besteht, welche bevorzugt miteinander verblockt sind. Insbesondere ist eine Heizstrecke 90 zum Erwärmen eines Behälter-Vorformlings (sogenannte Preform) vorgesehen, an welche sich ein Sterilisator 92 mit einer oder mehreren Behandlungseinheiten 920 anschließt, welcher zur Sterilisation der vorgeheizten Behälter-Vorformlinge dient. An den Sterilisator 92 schließt sich eine Streckblasvorrichtung 94 mit einem Streckblasrad 940 an, um die vorgeheizten und sterilisierten Vorformlinge in eine vorbestimmte Behältergeometrie zu bringen.

Nach Durchlaufen der Streckblasvorrichtung 94 sind die einzelnen Behälter 800 ausgeformt. Sowohl die Behälter-Vorformlinge als auch die Behälter 800 werden über unterschiedliche Behältertransporteinheiten 900 zwischen den jeweiligen aktiven Anlagenkomponenten transportiert.

Über diverse Behältertransporteinheiten 900, welche beispielsweise mit einer Inspektionsvorrichtung und einem Auswurf 910 zum Auswerfen fehlerhafter Behälter verbunden sind, werden die ausgeformten Behälter 800 nun zur eigentlichen Füllmaschine 96 gefördert, welche sich in einem Isolatorgehäuse mit einem isolierten Raum 7 befindet.

Eine Trennwand 912 und eine Schleuse 914 führen die Behälter in die Füllmaschine 96, in welcher ein Füllerkarussell 98 zum Befüllen der Behälter mit dem jeweiligen Getränk vorgesehen ist. An das Füllerkarussell 98 schließt sich der Verschließer 8 an, um die befüllten Behälter mit Verschlüssen 3 zu versehen. Eine Trennwand 980 trennt wiederum den aseptischen Reinraum 7 von dem Behälterauslauf 982, über welchen die nun befüllten und verschlossenen Behälter den Reinraum 7 verlassen.

Die Verschlüsse 3 werden über zwei Verschlussführungen 4, 5 in eine Behandlungskammer 2 eingeführt, wobei die Behandlungskammer 2 im Wesentlichen so ausgebildet ist, wie sie schematisch zu Figur 1 beschrieben ist.

Die Zuführung 840 der Verschlüsse 3 ist bevorzugt auf einem Podest erhöht über den restlichen Komponenten der Füllanlage 9 angeordnet, um im Bereich der Behandlungskammer 2 entsprechend ein schräges Herabrutschen der zu sterilisierenden Verschlüsse 3 zu erreichen.

Die unterschiedlichen Module der aseptischen Abfüllanlage 9, also der Sterilisator 92, die Streckblasvorrichtung 94, die Füllmaschine 96 sowie der Verschließer 8, sind bevorzugt in einem Isolator untergebracht, welcher mit einer Blecheinhausung 916 umgeben sein kann. Dabei sind, wie bereits beschrieben, einzelne Sektionen bevorzugt mit Trennwänden 912, 980 getrennt, um beispielsweise die Füllmaschine 96 von der Herstellung der Behälter lüftungstechnisch, beispielsweise über die Schleuse 914, zu trennen.

Die Transportvorrichtung 840 ist zweckmäßig, um so viele Verschlüsse 3 vorzuhalten, wie sich Behälter 800 beziehungsweise Vorformlinge in der Anlage befinden. Für diese Anzahl sind sämtliche Vorformlinge beziehungsweise Behälter 800 ab der Sperre 942 von Bedeutung.

Eine entsprechende intelligente Steuerung kann dabei Verluste von Vorformlingen und/oder Behältern 800 erkennen und auf Störungen im Zuführsystem beziehungsweise Abführsystem der Verschlüsse reagieren.

Die Vorformlinge sowie die Verschlüsse und auch die Gehäuse der Behälterbehandlungsanlage werden bevorzugt mit einem temperierten Wasserstoffperoxid (H₂O₂)-Luftgemisch (VHP = Vaporized Hydrogen Peroxide) sterilisiert.

Verschlüsse 3 sind bekannt, um Behälter 800 zu verschließen. Sie werden nicht nur mit unterschiedlichen Durchmessern bereitgestellt, sondern variieren auch bezüglich ihrer Form und der Ausbildung des verwendeten Materials. Auch können unterschiedliche Zusatzfunktionen, wie beispielsweise das Integrieren eines Trinkschnorchels, vorgesehen sein.

Die Vorrichtung 1 ist prinzipiell zur Behandlung sämtlicher Verschlüsse und Verschlusstypen geeignet. Um jedoch unterschiedlich breite beziehungsweise unterschiedlich hohe Verschlüsse zu behandeln, können auch unterschiedlich dimensionierte Verschlussführungen 4, 5 in der Behandlungskammer 2 vorgesehen werden.

In einer solchen Ausführungsform kann es zweckmäßig sein, die unterschiedlichen Verschlussführungen nicht mehr zusammenzuführen und jede Verschlussführung mit einem separaten Nockenrad derart zu versehen, dass die unterschiedlichen Verschlüsse quasi auf Knopfdruck dem Verschließer 8 zugeführt werden können.

Der Verschlussstrom selbst wird zum Anfahren der Anlage durch die Nockenräder in seiner Geschwindigkeit geregelt. Die durchschnittliche Behandlungszeit in der Behandlungskammer 2 liegt zwischen 6 und 21 Sekunden, je nach verwendetem Verschlusswerkstoff, verwendeter Verschlussgeometrie und geforderter Abtötungsrate.

Zum Abfahren der Anlage werden in einer weiteren Ausführungsform bevorzugt Verschlüsse in der Transportstrecke 840 beziehungsweise in der Behandlungskammer 2 vorgehalten. Der Verschlussstrom wird dabei über den Behälterstrom in der Anlage gesteuert, da sich der Behälterstrom nicht regeln lässt. Über die Nockenräder kann eine entsprechende Steuerung des Verschlussstromes jedoch einfach vorgenommen werden.

Figur 6 zeigt ein weiteres Ausführungsbeispiel einer Behandlungskammer 2, in welcher vier Verschlussführungen 4, 4', 5, 5' separat und nebeneinander durch die Behandlungskammer 2 geführt werden. Die Ansicht in Figur 6 blickt in Transportrichtung und die sich in der Mitte befindliche Verschlussführung entspricht der Führungsrinne 82 in Figur 4. Entsprechend werden die Verschlussführungen 4, 4', 5, 5' nach dem Durchlaufen der Behandlungsstrecke in einer Zusammenführvorrichtung 80 zusammengeführt, um dann dem Verschließer zugeführt zu werden.

Aus Figur 6 ist klar zu erkennen, dass jede der separat vorliegenden Verschlussschienen 4, 4', 5, 5' mit einer eigenen Steuervorrichtung mit eigenem Steuermotor 600 versehen ist, welcher separat die entsprechenden Nockenräder 604 so ansteuert, dass ein Zusammenführen der Verschlüsse 3 aus den nebeneinander durch die Behandlungskammer 2 geführten Verschlussführungen 4, 4', 5, 5' in die Führungsrinne 82 so durchgeführt werden kann, dass die Verschlüsse 3 immer abwechselnd zugeführt werden.

Durch das in Figur 6 gezeigte Bereitstellen von vier separat verlaufenden Verschlussführungen 4, 4', 5, 5', welche sich entlang des Transportpfades durch die Behandlungskammer 2 erstrecken, kann noch eine weitere Reduktion der Fließgeschwindigkeit der einzelnen Verschlüsse 3 durch die Behandlungskammer 2 erreicht werden. Auf diese Weise kann entweder der gesamte Durchsatz an Verschlüssen 3 erhöht werden, oder aber es kann die Behandlungslänge in der Behandlungskammer 2 noch weiter reduziert werden.

### Bezugszeichenliste

- 1: Vorrichtung zum Behandeln von Verschlüssen
- 2: Behandlungskammer
- 20: Anschlussflansch
- 22: stromaufwärts gelegenes Ende der Behandlungskammer
- 24: stromabwärts gelegenes Ende der Behandlungskammer
- 26, 28: Absaugvorrichtung
- 200: Düse für Sterilisationsmedium
- 210: Reinigungsdüsen
- 260, 280: Blenden
- 3: Verschluss
- 4, 4': Verschlussführung
- 40: drahtförmige Führung
- 42: Führungsrahmen
- 5, 5': Verschlussführung
- 60, 62: Steuervorrichtung am stromaufwärts gelegenen Ende der Behandlungskammer
- 64, 66: Steuervorrichtung am stromabwärts gelegenen Ende der Behandlungskammer
- 600: Steuermotor
- 602: Motorachse
- 604: Nockenrad
- 606: Nocke
- 7: steriler Anlagenraum
- 8: Verschließer
- 80: Zusammenführvorrichtung
- 82: Führungsrinne
- 84: Einteilrad
- 86: Düse
- 88: Verschlussstopper
- 800: Behälter
- 810: Verschließelement
- 840: Zuführung Verschlüsse
- 9: aseptische Abfüllanlage
- 90: Heizstrecke
- 92: Sterilisator
- 94: Streckblasvorrichtung
- 96: Füllmaschine
- 98: Füllerkarussell
- 900: Behältertransporteinheit
- 910: Auswurf
- 912: Trennwand
- 914: Schleuse
- 916: Blecheinhausung
- 920: Behandlungseinheit
- 940: Streckblasrad
- 942: Sperre
- 980: Trennwand
- 982: Behälterauslauf

- α: Winkel zwischen Verschlussführung und der Horizontalen

## Patentansprüche

1. Vorrichtung (1) zum Behandeln von Verschlüssen (3) für Behälter (800) umfassend eine Behandlungskammer (2) zum Behandeln der Verschlüsse (3) und einen Transportpfad zum Transportieren der Verschlüsse (3) durch die Behandlungskammer (2) hindurch, wobei der Transportpfad in der Behandlungskammer (2) mindestens zwei separat verlaufende Verschlussführungen (4, 5) zum gleichzeitigen Transport von Verschlüssen umfasst,
**dadurch gekennzeichnet, dass**
eine Steuervorrichtung zum Steuern des Verschlussstromes derart vorgesehen ist, dass die Verschlüsse (3) abwechselnd aus der ersten Verschlussführung (5) und der zweiten Verschlussführung (4) einem Verschließer (8) zuführbar sind.

2. Vorrichtung (1) gemäß Anspruch 1, **dadurch gekennzeichnet, dass** die mindestens zwei Verschlussführungen (4, 5) nebeneinander verlaufen, sich bevorzugt parallel zueinander erstrecken und besonders bevorzugt linear in der Behandlungskammer (2) verlaufen.

3. Vorrichtung (1) gemäß Anspruch 1 oder 2, **dadurch gekennzeichnet, dass** eine Vorrichtung zum Einbringen eines Sterilisationsmediums in die Behandlungskammer (2) vorgesehen ist, bevorzugt zum Einbringen von verdampftem Wasserstoffperoxid oder von Heißdampf.

4. Vorrichtung (1) gemäß einem der vorstehenden Ansprüche, **dadurch gekennzeichnet, dass** mindestens je eine der Verschlussführungen (4, 5) für mindestens einen ersten Verschluss und mindestens einen zweiten Verschluss vorgesehen sind, wobei der erste Verschluss und der zweite Verschluss unterschiedliche Dimensionen aufweisen, und bevorzugt der erste Verschluss eine Flat Cap und der zweite Verschluss eine Sport Cap sind.

5. Vorrichtung (1) gemäß einem der vorstehenden Ansprüche, **dadurch gekennzeichnet, dass** mindestens eine der Verschlussführungen (4, 5) eine drahtförmige Führung (40) aufweist, welche bevorzugt in einem Führungsrahmen (42) gehalten ist.

6. Vorrichtung (1) gemäß einem der vorstehenden Ansprüche, **dadurch gekennzeichnet, dass** die mindestens zwei Verschlussführungen (4, 5) so gegenüber der Horizontalen geneigt sind, dass die Verschlüsse (3) im Wesentlichen aufgrund der Gravitation durch die Behandlungskammer (2) transportiert werden, wobei die Verschlussführungen bevorzugt unter einem Winkel (α) von 20 und 45 Grad, besonders bevorzugt einem Winkel von 30 Grad, gegenüber der Horizontalen geneigt sind, und wobei die Verschlussführungen (4, 5) bevorzugt im Wesentlichen parallel zueinander verlaufen.

7. Vorrichtung (1) gemäß einem der vorstehenden Ansprüche, **dadurch gekennzeichnet, dass** mindestens eine weitere Steuervorrichtung (60, 62, 64, 66) zur Steuerung des Verschlussstromes in jeder Verschlussführung (4, 5) vorgesehen ist, und die Steuervorrichtung bevorzugt ein Nockenrad (604) zur Steuerung des Verschlussstromes aufweist.

8. Vorrichtung (1) gemäß Anspruch 7, **dadurch gekennzeichnet, dass** mindestens eine weitere erste Steuervorrichtung (60, 62) am stromaufwärts gelegenen Ende (22) der Behandlungskammer (2) vorgesehen ist, und mindestens eine weitere zweite Steuervorrichtung (64, 66) am stromabwärts gelegenen Ende (24) der Behandlungskammer (2) vorgesehen ist.

9. Vorrichtung (1) gemäß einem der vorstehenden Ansprüche, **dadurch gekennzeichnet, dass** vor mindestens einer Verschlussführung (4, 5) ein Sortierwerk, ein Verschlusspuffer und/oder eine Inspektionsvorrichtung zur Inspektion der Verschlüsse (3), bevorzugt bezüglich deren Lage und Form, vorgesehen ist.

10. Vorrichtung (1) gemäß einem der vorstehenden Ansprüche, **dadurch gekennzeichnet, dass** mindestens eine Absaugkammer (26, 28) zum Absaugen des Sterilisationsmediums an mindestens einem Ende der Behandlungskammer (2) vorgesehen ist, um ein Austreten von Sterilisierungsmedium aus der Behandlungskammer (2) zu reduzieren oder zu verhindern, wobei bevorzugt eine erste Absaugkammer (26) am stromaufwärts gelegenen Ende (22) der Behandlungskammer (2) angeordnet ist, und bevorzugt mindestens eine zweite Absaugkammer (28) am stromabwärts gelegenen Ende (24) der Behandlungskammer (2) angeordnet ist.

11. Vorrichtung (1) gemäß einem der vorstehenden Ansprüche, **dadurch gekennzeichnet, dass** die mindestens zwei Verschlussführungen (4, 5) am stromabwärts gelegenen Ende (24) der Behandlungskammer (2) zu einer einzigen Führungsrinne (82) zusammengeführt sind.

12. Verfahren zum Behandeln von Verschlüssen (3) für Behälter (800), umfassend die Schritte:
Transportieren der Verschlüsse (3) entlang eines Transportpfades durch eine Behandlungskammer (2) hindurch, um die Verschlüsse (3) zu behandeln, wobei mindestens zwei Verschlüsse (3) auf separaten Verschlussführungen gleichzeitig durch die Behandlungskammer (2) transportiert werden,
**dadurch gekennzeichnet, dass**
eine Steuervorrichtung zum Steuern des Verschlussstromes derart vorgesehen ist, dass die Verschlüsse (3) abwechselnd aus der ersten Verschlussführung (5) und der zweiten Verschlussführung (4) einem Verschließer (8) zugeführt werden.

13. Verfahren gemäß Anspruch 12, **dadurch gekennzeichnet, dass** die Verschlüsse (3) in der Behandlungskammer (2) mit einem Sterilisationsmedium behandelt werden, bevorzugt mit verdampftem Wasserstoffperoxid oder mit Heißdampf.

14. Verfahren gemäß Anspruch 12 oder 13, **dadurch gekennzeichnet, dass** mindestens zwei Verschlüsse auf nebeneinander angeordneten Verschlussführungen, bevorzugt auf parallel zueinander angeordneten Verschlussführungen, durch die Behandlungskammer (2) transportiert werden.

## Claims

1. Apparatus (1) for treating closures (3) for containers (800), comprising a treatment chamber (2) for treating the closures (3) and a transporting path for transporting the closures (3) through the treatment chamber (2), wherein the transporting path, in the treatment chamber (2), comprises at least two separately running closure guides (4, 5) for transporting closures simultaneously,
**characterized in that**
a control arrangement for controlling the stream of closures is provided such that the closures (3) can be fed to a capper (8) alternately from the first closure guide (5) and the second closure guide (4).

2. Apparatus (1) according to claim 1, **characterized in that** the at least two closure guides (4, 5) run alongside one another, preferably extend parallel to one another and more preferably run linearly in the treatment chamber (2).

3. Apparatus (1) according to claim 1 or 2, **characterized in that** an arrangement for introducing a sterilization medium into the treatment chamber (2) is provided, preferably for introducing evaporated hydrogen peroxide or superheated steam.

4. Apparatus (1) according to any of the preceding claims, **characterized in that** for each of the at least one first closure and at least one second closure at least one of the closure guides (4, 5) is provided, wherein the first closure and the second closure comprise different dimensions, and preferably the first closure is a flat cap and the second closure is a sport cap.

5. Apparatus (1) according to any of the preceding claims, **characterized in that** at least one of the closure guides (4, 5) comprises a wire-like guide (40), which is retained preferably in a guide frame (42).

6. Apparatus (1) according to any of the preceding claims, **characterized in that** the at least two closure guides (4, 5) are inclined in relation to the horizontal such that the closures (3) are transported through the treatment chamber (2) substantially due to gravity, wherein the closure guides are inclined preferably at an angle (α) of between 20 and 45 degrees, more preferably an angle of 30 degrees, in relation to the horizontal, and wherein the closure guides (4, 5) preferably run substantially parallel to one another.

7. Apparatus (1) according to any of the preceding claims, **characterized in that** at least one further control arrangement (60, 62, 64, 66) for controlling the stream of closures is provided in each closure guide (4, 5), and the control arrangement preferably has a cam wheel (604) for controlling the stream of closures.

8. Apparatus (1) according to claim 7, **characterized in that** at least one further first control arrangement (60, 62) is provided at the upstream end (22) of the treatment chamber (2), and at least one further second control arrangement (64, 66) is provided at the downstream end (24) of the treatment chamber (2).

9. Apparatus (1) according to any of the preceding claims, **characterized in that** upstream of at least one closure guide (4, 5), a sorting unit, a closure buffer, and/or an inspection means for inspecting the closures (3) is provided, preferably in respect of their position and shape.

10. Apparatus (1) according to any of the preceding claims, **characterized in that** at least one suction-extraction chamber (26, 28) for extracting by suction the sterilization medium is provided at at least one end of the treatment chamber (2), in order to reduce or prevent exiting of sterilization medium from the treatment chamber (2), wherein preferably a first suction-extraction chamber (26) is arranged at the upstream end (22) of the treatment chamber (2), and preferably at least a second suction-extraction chamber (28) is arranged at the downstream end (24) of the treatment chamber (2).

11. Apparatus (1) according to any of the preceding claims, **characterized in that** the at least two closure guides (4, 5) are brought together at the downstream end (24) of the treatment chamber (2) to form a single guide channel (82).

12. Method of treating closures (3) for containers (800), comprising the following steps:
transporting the closures (3), along a transporting path, through a treatment chamber (2) in order to treat the closures (3), wherein at least two closures (3) are transported simultaneously, on separate closure guides, through the treatment chamber (2),
**characterized in that**
a control means for controlling the stream of closures is provided such that the closures (3) are fed to a capper (8) alternately from the first closure guide (5) and the second closure guide (4).

13. Method according to claim 12, **characterized in that** the closures (3) are treated in the treatment chamber (2) with a sterilization medium, preferably with evaporated hydrogen peroxide or with superheated steam.

14. Method according to claim 12 or 13, **characterized in that** at least two closures are transported through the treatment chamber (2) on closure guides arranged alongside one another, preferably on closure guides arranged parallel to one another.

## Revendications

1. Dispositif (1) pour traiter des bouchons (3) pour des récipients (800) comprenant une chambre de traitement (2) pour traiter les bouchons (3) et un chemin de transport pour transporter les bouchons (3) à travers la chambre de traitement (2), le chemin de transport dans la chambre de traitement (2) comprenant au moins deux guides de bouchons s'étendant séparément (4, 5) pour le transport simultané de bouchons,
**caractérisé en ce**
**qu'**un dispositif de commande pour commander le flux de bouchons est prévu de telle sorte que les bouchons (3) puissent être acheminés en alternance depuis le premier guide de bouchons (5) et le deuxième guide de bouchons (4) à un dispositif de fermeture (8).

2. Dispositif (1) selon la revendication 1, **caractérisé en ce que** les au moins deux guides de bouchons (4, 5) s'étendent l'un à côté de l'autre, s'étendent de préférence parallèlement l'un à l'autre et particulièrement préférablement linéairement dans la chambre de traitement (2).

3. Dispositif (1) selon la revendication 1 ou 2, **caractérisé en ce qu'**il est prévu un dispositif pour introduire un milieu de stérilisation dans la chambre de traitement (2), de préférence pour introduire du peroxyde d'hydrogène évaporé ou de la vapeur chaude.

4. Dispositif (1) selon l'une quelconque des revendications précédentes, **caractérisé en ce qu'**au moins à chaque fois l'un des guides de bouchons (4, 5) est prévu respectivement pour au moins un premier bouchon et au moins un deuxième bouchon, le premier bouchon et le deuxième bouchon présentant des dimensions différentes, et de préférence le premier bouchon étant un bouchon plat et le deuxième bouchon étant un bouchon "sport".

5. Dispositif (1) selon l'une quelconque des revendications précédentes, **caractérisé en ce qu'**au moins l'un des guides de bouchons (4, 5) présente un guide en forme de fil (40) qui est retenu de préférence dans un cadre de guidage (42).

6. Dispositif (1) selon l'une quelconque des revendications précédentes, **caractérisé en ce que** les au moins deux guides de bouchons (4, 5) sont inclinés par rapport à l'horizontale de telle sorte que les bouchons (3) soient transportés essentiellement sous l'effet de la gravité à travers la chambre de traitement (2), les guides de bouchons étant inclinés de préférence suivant un angle (α) d'entre 20 et 45°, particulièrement préférablement suivant un angle de 30°, par rapport à l'horizontale et les guides de bouchons (4, 5) s'étendant de préférence essentiellement parallèlement l'un à l'autre.

7. Dispositif (1) selon l'une quelconque des revendications précédentes, **caractérisé en ce qu'**au moins un dispositif de commande supplémentaire (60, 62, 64, 66) est prévu pour commander le flux de bouchons dans chaque guide de bouchons (4, 5) et le dispositif de commande présente de préférence une roue à came (604) pour commander le flux de bouchons.

8. Dispositif (1) selon la revendication 7, **caractérisé en ce qu'**au moins un premier dispositif de commande supplémentaire (60, 62) est prévu à l'extrémité amont (22) de la chambre de traitement (2) et au moins un deuxième dispositif de commande supplémentaire (64, 66) est prévu à l'extrémité aval (24) de la chambre de traitement (2).

9. Dispositif (1) selon l'une quelconque des revendications précédentes, **caractérisé en ce qu'**avant au moins un guide de bouchons (4, 5) est prévu un mécanisme de triage, un tampon de bouchons et/ou un dispositif d'inspection pour inspecter les bouchons (3), de préférence en termes de leur position et de leur forme.

10. Dispositif (1) selon l'une quelconque des revendications précédentes, **caractérisé en ce qu'**au moins une chambre d'aspiration (26, 28) pour aspirer le milieu de stérilisation est prévue au niveau d'au moins une extrémité de la chambre de traitement (2), afin de réduire ou d'empêcher une fuite de milieu de stérilisation hors de la chambre de traitement (2), de préférence une première chambre d'aspiration (26) étant disposée à l'extrémité amont (22) de la chambre de traitement (2) et de préférence au moins une deuxième chambre d'aspiration (28) étant disposée à l'extrémité aval (24) de la chambre de traitement (2).

11. Dispositif (1) selon l'une quelconque des revendications précédentes, **caractérisé en ce que** les au moins deux guides de bouchons (4, 5) sont réunis à l'extrémité aval (24) de la chambre de traitement (2) pour former une rigole de guidage unique (82).

12. Procédé de traitement de bouchons (3) pour des récipients (800), comprenant les étapes suivantes :
transporter les bouchons (3) le long d'un chemin de transport à travers une chambre de traitement (2), afin de traiter les bouchons (3), au moins deux bouchons (3) étant transportés simultanément à travers la chambre de traitement (2) sur des guides de bouchons séparés,
**caractérisé en ce**
**qu'**un dispositif de commande pour commander le flux de bouchons est prévu de telle sorte que les bouchons (3) soient acheminés en alternance depuis le premier guide de bouchons (5) et le deuxième guide de bouchons (4) à un dispositif de fermeture (8).

13. Procédé selon la revendication 12, **caractérisé en ce que** les bouchons (3) sont traités dans la chambre de traitement (2) avec un milieu de stérilisation, de préférence avec du peroxyde d'hydrogène évaporé ou avec de la vapeur chaude.

14. Procédé selon la revendication 12 ou 13, **caractérisé en ce qu'**au moins deux bouchons sont transportés à travers la chambre de traitement (2) sur des guides de bouchons disposés l'un à côté de l'autre, de préférence sur des guides de bouchons disposés parallèlement l'un à l'autre.
